(19) Europäisches Patentamt
European Patent Office
Office européen des brevets

(11) Veröffentlichungsnummer: **0 016 978 B1**

(12) **EUROPÄISCHE PATENTSCHRIFT**

(45) Veröffentlichungstag der Patentschrift: **13.05.87**

(21) Anmeldenummer: **80101059.6**

(22) Anmeldetag: **03.03.80**

(51) Int. Cl.⁴: **C 07 C 120/00,** C 07 C 121/66, C 07 C 121/68, C 07 D 213/57, C 07 D 215/12 // C07C79/35, C07C87/457, C07C93/14, C07C101/28, C07D213/38, C07D295/02

# (54) Verfahren zur Herstellung von Nitrilen.

(30) Priorität: **09.03.79 DE 2909871**

(43) Veröffentlichungstag der Anmeldung: **15.10.80 Patentblatt 80/21**

(45) Bekanntmachung des Hinweises auf die Patenterteilung: **13.05.87 Patentblatt 87/20**

(84) Benannte Vertragsstaaten: **AT BE CH DE FR GB IT LU NL SE**

(56) Entgegenhaltungen:
**HELVETICA CHIMICA ACTA, Band 59, Heft 8, 1976 Basel. J.STREITH et al. "L'acide hydroxylamine-O-sulfonique, réactif de choix pour la conversion d'aldéhydes en nitriles" Seiten 2786 bis 2792**
**Norman L. Allinger u.a., Organic Chemistry 1971, Seite 583.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Biere, Helmut, Dr., Zeltinger Strasse 15, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 016 978 B1

**Beschreibung**

Die Erfindung betrifft ein Verfahren zur Herstellung von Nitrilen der allgemeinen Formel I

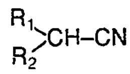 (I),

worin

$R_1$ eine gegebenenfalls durch niedere Alkylgruppen mit 1 bis 4 Kohlenstoffatomen, Phenylgruppen, Niederalkoxygruppen mit 1 bis 4 Kohlenstoffatomen, Phenoxygruppen, Halogenatome, Nitrogruppen, Cyanogruppen, Carboxylgruppen oder Niederalkoxycarbonylgruppen mit 1 bis 4 Kohlenstoffatomen im Alkoxyrest substituierte Phenylgruppe, Indanylgruppe, Naphthylgruppe, Pyridylgruppe, Indolylgruppe, Chinazolinylgruppe, Chinolylgruppe oder Thienylgruppe und $R_2$ ein Wasserstoffatom, eine niedere Alkylgruppe eine Niederalkoxycarbonylgruppe oder eine mit $R_1$ verknüpfte Dimethylengruppe bedeuten, das dadurch gekennzeichnet ist, dass man ein Enamin der allgemeinen Formel II

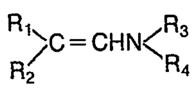 (II),

worin

$R_1$ und $R_2$ die obengenannte Bedeutung besitzen und $R_3$ und $R_4$ jeweils niedere Alkylgruppen oder gemeinsam eine gegebenenfalls durch ein Sauerstoffatom oder eine Niederalkyliminogruppe unterbrochene Tetramethylengruppe oder Pentamethylengruppe darstellen, in Gegenwart von Wasser bei einem pH-Wert von 0 bis 3 mit Hydroxylamin-O-sulfonsäure umsetzt.

Nitrile sind bekanntlich wichtige Zwischenprodukte zur Synthese von Carbonsäuren, Estern, Amiden, Aldehyden, Aminen etc.. Sie sind demzufolge als Rohstoffe für die Synthese von Schädlingsbekämpfungsmitteln, Farbstoffpigmenten, Pharmazeutika etc. von grosser Bedeutung. Ihre technische Herstellung erfolgt meist nach dem Verfahren der sogenannten Kolbschen Nitrilsynthese durch Einwirkung von Alkali- oder Erdalkalicyanid auf die entsprechenden Halogenide.

Da die bei der Kolbschen Nitrilsynthese meist verwendeten Alkalicyanide besonders toxisch sind, müssen besondere Arbeitsschutzmassnahmen getroffen und aufwendige Abwasserreinigungs- bzw. -aufbereitungsmassnahmen angeschlossen werden. Hinzu kommt, dass als Nebenreaktion zur Nitrilbildung oft die Bildung von Isonitrilen beobachtet wird, die äusserst geruchsbelästigend wirken. Ein sehr ernster weiterer Nachteil dieser Synthese ist, dass viele, besonders heterocyclische Alkylhalogenide aufgrund der Basizität der verwendeten Alkalicyanide mehrfach substituierte Nitrile oder deren Zersetzungsprodukte liefern. In einigen Fällen hat man durch Arbeiten in aprotischen Lösungsmitteln und mit Zusätzen von Kronenäthern bzw. durch die Phasentransfertechnik Abhilfe schaffen können, jedoch wird die Synthese durch die Anwendung dieser Stoffe für eine technische Anwendung meist zu aufwendig.

Es ist bekannt (Helv. Chim. Acta 59, (1976), 2786–2792), dass man Aldehyde mittels Hydroxylamino-O-sulfonsäure in Nitrile überführen kann, die hierzu benötigten Aldehyde sind aber von wenigen Ausnahmen abgesehen sehr oxidationsempfindlich und/oder neigen zur Polymerisation und sind demzufolge technisch weniger gut handhabbar.

Demgegenüber ist das erfindungsgemässe Verfahren in einfacher Weise durchführbar und mit den obengenannten Nachteilen der Kolbeschen Nitrilsynthese nicht behaftet.

Die für das erfindungsgemässe Verfahren verwendeten Enamine der allgemeinen Formel II können als Substituenten $R_1$ mono- oder bicyclische, aromatische oder heterocyclische Reste tragen. Geeignete Reste $R_1$ sind beispielsweise der Phenylrest, der Indanylrest oder der α- oder β-Naphthylrest. Geeignete heterocyclische Reste $R_1$ sind beispielsweise der Thienylrest, der Indolrest, der Pyridinrest oder der Chinolinrest.

Der aromatische oder heterocyclische Rest $R_1$ kann unsubstituiert oder substituiert sein. Geeignete Substituenten des Restes $R_1$ sind niedere Alkylgruppen mit 1 bis 4 Kohlenstoffatomen (z.B. die Methylgruppe, die Ethylgruppe, die Propylgruppe, die Isopropylgruppe oder die tert.-Butylgruppe), Phenylgruppen, Niederalkoxygruppen mit 1 bis 4 Kohlenstoffatomen im Alkoxyrest (z.B. die Methoxygruppe, die Ethoxygruppe, die Propyloxygruppe, die Isopropyloxygruppe oder die tert.-Butyloxygruppe), Phenoxygruppen, Halogenatome (Fluor-, Chlor-, Brom- oder Jodatome), Nitrogruppen, Cyanogruppen, Carboxylgruppen oder Niederalkoxycarbonylgruppen mit 1 bis 4 Kohlenstoffatomen im Alkoxyrest (z.B. die Methoxycarbonylgruppe, die Äthoxycarbonylgruppe, die Propyloxycarbonylgruppe, die Isopropyloxycarbonylgruppe oder die Butyloxycarbonylgruppe.

Das erfindungsgemässe Verfahren wird in Gegenwart von Wasser durchgeführt. Das Wasser selbst kann als Lösungsmittel dienen. Andererseits ist es aber auch möglich, die Reaktion in Gemischen von polaren inerten Lösungsmitteln mit Wasser durchzuführen. Solche inerten polaren Lösungsmittel sind beispielsweise niedere Alkohole (Methanol, Äthanol, Isopropanol etc.), niedere Carbonsäuren (vorzugsweise Essigsäure), polare Äther (wie Glykolmonomethyläther, Glykoldimethyläther, Tetrahydrofuran oder Dioxan) oder dipolare aprotische Lösungsmittel (wie Dimethylformamid, N-Methylacetamid, Dimethylsulfoxid oder Hexamethylphosphorsäuretriamid).

Das erfindungsgemässe Verfahren wird bei einem pH-Wert von 0 bis 3, vorzugsweise 0 bis 1, durchgeführt. Die Einstellung des sauren Reaktionsmilieus kann in der Weise erfolgen, dass man die Hydroxylamin-O-sulfonsäure im Überschuss anwendet (vorzugsweise 1,5 mol bis 5 mol Hydroxylamin-O-sulfonsäure pro mol Enamin). Andererseits ist es aber auch möglich, mit etwa äquimolaren Mengen an Hydroxylamin-O-sulfonsäure zu arbeiten, wenn man dem Reaktionsgemisch zusätzlich noch Mineralsäuren (Salzsäure,

Schwefelsäure, Phosphorsäure, Perchlorsäure etc.) zusetzt.

Die Reaktion erfolgt vorzugsweise bei einer Reaktionstemperatur von 0 bis 100 °C; die Reaktionszeit beträgt in der Regel 2 bis 24 Stunden.

Die Ausgangsenamine der Formel II sind literaturbekannt oder werden in an sich bekannter Weise auf verschiedenen, dem Fachmann geläufigen Wegen hergestellt (z.B. Enamines, G. Cook, Marcel-Dekker-Verlag, New York and London, 1969, Chem. Ber. 101, 4048, 1968, Liebigs Ann. 641, 1961).

Die nachfolgenden Ausführungsbeispiele dienen zur Erläuterung des erfindungsgemässen Verfahrens.

Beispiel 1

a) Zu einer Lösung von 850 mg Hydroxylamin-O-sulfonsäure in 15 ml Wasser werden 660 mg 1-Dimethyl-aminomethylen-5-nitroindan hinzugefügt und die Mischung 2 Stunden bei Raumtemperatur gerührt. Das entstehende Kristallisat wird abgesaugt und mit Wasser gewaschen. Nach Umkristallisieren aus Äthanol erhält man 510 mg (90%) 5-Nitroindan-1-carbonitril mit Schmelzpunkt 117 °C.

b) Zur Darstellung des Ausgangsmaterials wird folgendermassen verfahren:

Ein Gemisch von 820 mg 5-Nitroindan und 1,7 g Bis(dimethylamino)-tert.-butoxymethan (dargestellt nach H. Bredereck et al., Chem Ber. 101, 41 (1968)) wird auf 160 °C (Badtemperatur) erhitzt und der entstehende tert.-Butylalkohol über eine Kolonne abdestilliert. Nach einer Stunde wird das dunkelrote Reaktionsgemisch abgekühlt und aus Äthanol kristallisiert. Man erhält 775 mg (71%) 1-Dimethylaminomethylen-5-nitroindan mit Schmelzpunkt 121 °C.

Beispiel 2

Analog Beispiel 1 wird α-Styrylmorpholin (dargestellt aus Phenylacetaldehyd und Morpholin) mit Hydroxylamin-O-sulfonsäure umgesetzt. Nach Extraktion der wässrigen Phase mit Äthylacetat wird eingeengt und der Rückstand im Vakuum destilliert. Man erhält 70% Phenylacetonitril mit $n_D^{23}$ 1.5190.

Beispiel 3

a) Analog Beispiel 1 wird α-Dimethylamino-4-nitrostyrol umgesetzt. Nach Umkristallisieren aus Äthanol erhält man 78% 4-Nitrophenylacetonitril mit Schmelzpunkt 116 °C.

b) Das 4-Nitrophenylacetonitril kann auch auf folgende Weise erhalten werden:

Zu einer Mischung von 420 mg Hydroxylamin-O-sulfonsäure und 1 ml 2 N Schwefelsäure in 15 ml Wasser werden 580 mg α-Dimethylamino-4-nitrostyrol zugefügt und 1 Stunde bei Raumtemperatur gerührt. Nach Absaugen des Niederschlages wird aus Äthanol umkristallisiert; man erhält 400 mg (82%) mit Schmelzpunkt 115 °C.

c) Das literaturbekannte α-Dimethylamino-4-nitrostyrol wird aus 4-Nitrotoluol analog Beispiel 1 b hergestellt.

Beispiel 4

a) Analog Beispiel 1 a wird 1-Dimethylamino-2-(4-nitrophenyl)-1-propen mit Hydroxylamin-O-sulfonsäure umgesetzt und man erhält nach Umkristallisieren aus Äthanol 80% 2-(4-Nitrophenyl)-propionitril mit Schmelzpunkt 76 °C.

b) Das Ausgangsmaterial wird analog Beispiel 1 b aus 1-Äthyl-4-nitrobenzol hergestellt. Man erhält nach Umkristallisieren aus Äthanol 66% 1-Dimethylamino-2-(4-nitrophenyl)-propen mit Schmelzpunkt 76 °C.

Beispiel 5

a) Analog Beispiel 1 a wird 4-(2-Dimethylamino-vinyl)-pyridin mit Hydroxylamin-O-sulfonsäure umgesetzt. Nach beendeter Reaktion wird die Lösung auf pH-12 eingestellt und mit Äthylacetat extrahiert. Der organische Rückstand wird über Kieselgel mit Toluol/Äthylacetat chromatographiert und anschliessend aus Äthylacetat/Hexan umkristallisiert. Man erhält 68% 4-Pyridyl-acetonitril mit Schmelzpunkt 43 °C.

b) Analog Beispiel 1 b wird das 4-(2-Dimethyl-aminovinyl)-pyridin aus 4-Picolin hergestellt, Schmelzpunkt 103 °C.

Beispiel 6

a) Analog Beispiel 1 a wird 3-Dimethylamino-2-phenyl-acrylsäure-äthylester mit Hydroxylamin-O-sulfonsäure umgesetzt. Nach Extrahieren mit Äthylacetat wird 2-Cyano-phenylessigsäure-äthylester (82%) erhalten, der durch Vakuumdestillation weiter aufgereinigt werden kann.

b) Das Ausgangsmaterial wird analog Beispiel 1 b aus Phenylessigsäure-äthylester und Bis(dimethylamino)tert.-butoxymethan gewonnen. Nach Vakuumdestillation des Rückstandes ($Kp_{0.01}$ 135–40 °C) erhält man 3-Dimethylamino-2-phenyl-acrylsäure-äthylester mit $n_D^{23}$ 1.5682.

Beispiel 7

a) Analog Beispiel 1 a wird 1-Dimethylamino-methylen-5-nitro-6-phenoxyindan in Waser unter Zusatz von Dioxan mit Hydroxylamin-O-sulfonsäure zu 5-Nitro-6-phenoxy-indan-1-carbonitril mit Schmelzpunkt 78 °C (Isopropylalkohol) umgesetzt.

b) Das Ausgangsmaterial wird analog Beispiel 1 b aus 5-Nitro-6-phenoxyindan durch Reaktion mit Bis(dimethylamino)tert.-butoxymethan gewonnen. Man erhält nach Kristallisation aus Äthanol 1-Dimethylaminomethylen-5-nitro-6-phenoxyindan mit Schmelzpunkt 97 °C.

c) 5-Nitro-6-phenoxyindan wird auf folgende Weise erhalten:

12,1 g 6-Brom-5-nitroindan, 0,75 g Kupfer(I)chlorid, 17,3 g Kaliumcarbonat und 18,4 g Phenol in 200 ml absolutem Pyridin werden 3 Stunden am Rückfluss gekocht. Man engt im Vakuum ein, versetzt den Rückstand mit Chloroform und saugt vom Unlöslichen ab. Die Mutterlauge wird mit 1 N Natronlauge und 1 N Salzsäure ausgeschüttelt und im Vakuum eingeengt. Nach Destillation im Vakuum werden 11,5 g 5-Nitro-6-phenoxyindan vom Siedepunkt $Kp_{0.03}$ 163–165 °C erhalten.

Beispiel 8

a) Analog Beispiel 1 a wird 2-Chlor-4-(2-dimethylaminovinyl)-8-phenylchinolin mit Hydroxylamin-O-sulfonsäure bei Raumtemperatur umgesetzt. Man erhält nach Umkristallisation aus Äthanol 2-Chlor-8-phenyl-4-chinolinyl-acetonitril mit Schmelzpunkt 176 °C.

b) Analog Beispiel 1 b wird 2-Chlor-4-methyl-8-phenylchinolin (dargestellt aus 2-Hydroxy-4-methyl-8-phenylchinolin) mit Bis(dimethylamino)tert.-butoxymethan umgesetzt zu 2-Chlor-4-(2-dimethylaminovinyl)-8-phenylchinolin mit Schmelzpunkt 150 °C. (Äthylacetat)

**Patentansprüche**

1. Verfahren zur Herstellung von Nitrilen der allgemeinen Formel I

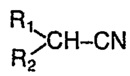 (I),

worin

$R_1$ eine gegebenenfalls durch niedere Alkylgruppen mit 1 bis 4 Kohlenstoffatomen, Phenylgruppen, Niederalkoxygruppen mit 1 bis 4 Kohlenstoffatomen, Phenoxygruppen, Halogenatome, Nitrogruppen, Cyanogruppen, Carboxylgruppen oder Niederalkoxycarbonylgruppen mit 1 bis 4 Kohlenstoffatomen im Alkoxyrest substituierte Phenylgruppe, Indanylgruppe, Naphtylgruppe, Pyridylgruppe, Indolylgruppe, Chinazolinylgruppe, Chinolylgruppe oder Thienylgruppe und $R_2$ ein Wasserstoffatom, eine niedere Alkylgruppe, eine Niederalkoxycarbonylgruppe oder eine mit $R_1$ verknüpfte Dimethylengruppe bedeuten, dadurch gekennzeichnet, dass man ein Enamin der allgemeinen Formel II

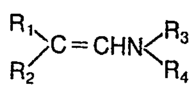 (II),

worin

$R_1$ und $R_2$ die obengenannte Bedeutung besitzen und $R_3$ und

$R_4$ jeweils niedere Alkylgruppen

oder gemeinsam eine gegebenenfalls durch ein Sauerstoffatom oder eine Niederalkyliminogruppe unterbrochene Tetramethylengruppe oder Pentamethylengruppe darstellen, in Gegenwart von Wasser bei einem pH-Wert von 0 bis 3 mit Hydroxylamin-O-sulfonsäure umsetzt.

2. Verfahren zur Herstellung von Nitrilen der allgemeinen Formel I gemäss Anspruch 1, worin

$R_1$ eine gegebenenfalls durch eine Nitrogruppe, ein Halogenatom, eine Phenylgruppe, eine Phenoxygruppe substituierte Phenylgruppe, Pyridylgruppe oder Chinolylgruppe und

$R_2$ ein Wasserstoffatom, eine Methylgruppe, eine Niederalkoxycarbonylgruppe oder eine mit $R_1$ verknüpfte Dimethylengruppe bedeuten.

**Claims**

1. Process for the preparation of nitriles of general formula I

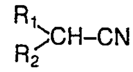 (I),

wherein

$R_1$ is a phenyl, indanyl, naphthyl, pyridyl, indolyl, quinazolinyl, quinolyl or a thienyl group, said groups being optionally substituted by alkyl groups of 1 to 4 carbon atoms, phenyl groups, lower alkoxy groups of 1 to 4 carbon atoms, phenoxy groups, halogen atoms, nitro groups, cyano groups, carboxyl groups or lower alkoxycarbonyl groups, with 1 to 4 carbon atoms in the alkoxy group, and $R_2$ is a hydrogen atom, a lower alkyl group, a lower alkoxycarbonyl group or a dimethylene group linked to $R_1$, characterised in that an enamine of general formula II

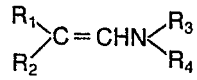 (II),

wherein

$R_1$ and $R_2$ have the meanings given above and $R_3$ and $R_4$ are each lower alkyl groups or together form a tetramethylene or pentamethylene group, optionally interrupted by an oxygen or a lower alkylamino group, is reacted with hydroxylamine-O-sulphonic acid, in the presence of water at a pH of 0 to 3.

2. Process for the preparation of nitriles of general formula I according to claim 1, wherein

$R_1$ is a phenyl, pyridyl or quinolyl group, said groups being optionally substituted by a nitro group, a halogen atom, a phenyl group or a phenoxy group and

$R_2$ is a hydrogen atom, a methyl group, a lower alkoxycarbonyl group or a dimethylene group linked to $R_1$.

**Revendications**

1. Procédé de préparation de nitriles répondant à la formule générale I:

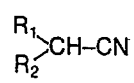 (I)

dans laquelle:

$R_1$ représente un radical phényle éventuellement porteur de radicaux alkyles inférieurs contenant de 1 à 4 atomes de carbone, de radicaux phényles, de radicaux alcoxy inférieurs contenant de 1 à 4 atomes de carbone, de radicaux phénoxy, d'atomes d'halogènes, de radicaux nitro, de radicaux cyano, de radicaux carboxy ou de radicaux alcoxycarbonyles inférieurs dont la partie alcoxy contient de 1 à 4 atomes de carbone, ou un radical indanyle, naphtyle, pyridyle, indolyle, quinazolinyle, quinolyle ou thiényle, et

$R_2$ représente un atome d'hydrogène, un radical alkyle inférieur, un radical alcoxycarbonyle inférieur ou un radical diméthylène lié à $R_1$,
procédé caractérisé en ce qu'on fait réagir une énamine répondant à la formule générale II:

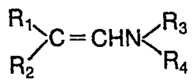

(II)

dans laquelle:
$R_1$ et $R_2$ ont les significations précédemment données et

$R_3$ et $R_4$ représentent chacun un radical alkyle inférieur ou forment ensemble un radical tétraméthylène ou pentaméthylène éventuellement interrompu par un atome d'oxygène ou par un radical alkylimino inférieur,
en présence d'eau, à un pH de 0 à 3, avec l'acide hydroxylamine-O-sulfonique.

2. Procédé selon la revendication 1 caractérisé en ce qu'on prépare des nitriles de formule générale I dans lesquels:

$R_1$ représente un radical phényle éventuellement porteur d'un radical nitro, d'un atome d'halogène, d'un radical phényle ou d'un radical phénoxy, un radical pyridyle ou un radical quinolyle et

$R_2$ représente un atome d'hydrogène, un radical méthyle, un radical alcoxycarbonyle inférieur ou un radical diméthylène lié à $R_1$.